# EUROPEAN PATENT SPECIFICATION

(11) **EP 4 236 705 B1**
(45) Date of publication and mention of the grant of the patent: **25.02.2026**
(21) Application number: 21890019.9
(22) Date of filing: 03.11.2021
(51) Int. Cl.: A24F 40/10, A61M 15/06, A24F 40/46, A24F 40/05, A24F 40/42, A61M 15/00, B05B 17/00, B05B 17/06, A61M 11/00

(54) **ELECTRONIC DEVICES FOR AEROSOLIZING AND INHALING LIQUID**
ELEKTRONISCHE VORRICHTUNGEN ZUR AEROSOLISIERUNG UND INHALATION VON FLÜSSIGKEIT
DISPOSITIFS ÉLECTRONIQUES POUR L'AÉROSOLISATION ET L'INHALATION DE LIQUIDE

(30) Priority: 03.11.2020 US 202063108880 P
(43) Date of publication of application: 06.09.2023
(73) Proprietor: Qnovia, Inc., Richmond VA 23230 (US)
(72) Inventor: DANEK, Mario, Los Angeles, CA 90048 (US); KOVACEVICH, Ian, Carlsbad, CA 92008 (US); BETTS, Kassie, San Diego, CA 92130 (US)
(74) Representative: Swindell & Pearson Limited
(86) International application number: PCT/US2021/057963
(87) International publication number: WO 2022/098802

(56) References cited:
- WO-A1-2019/239217
- WO-A1-2020/234807
- US-A- 5 894 841
- US-A1- 2003 192 959
- US-A1- 2016 228 658
- US-A1- 2016 228 658
- US-A1- 2019 124 992
- US-A1- 2020 060 349
- US-A1- 2020 120 989
- US-A1- 2020 230 329
- US-B2- 10 575 564

## Description

### COPYRIGHT STATEMENT

All of the material in this patent document is subject to copyright protection under the copyright laws of the United States and other countries. The copyright owner has no objection to the facsimile reproduction by anyone of the patent document or the patent disclosure, as it appears in official governmental records but, otherwise, all other copyright rights whatsoever are reserved.

### BACKGROUND OF THE INVENTION

The invention generally relates to apparatus, and systems for producing an aerosol for inhalation by a person, whether intended for personal or recreational use, or for the administration of medicines.

Vaping has been rapidly increasing in popularity, primarily because vaping provides a convenient, discreet, and presumably benign way to self-administer nicotine, cannabis, drugs, or other micronutrients. Indeed, there is a common belief that vaping is healthier than smoking cigarettes; vaping purportedly lets smokers avoid dangerous chemicals inhaled from regular cigarettes while still getting nicotine. Vaping also can be used for cannabis.

Vaping is performed using a vaporizer. A vaporizer includes a vape pen or a cigarette style vape, referred to by many as an e-cigarette or "eCig". A vape pen generally is an elongate, thin, and stylized tube that resembles a fancy pen. In contrast, an e-cigarette resembles an actual cigarette. The e-cigarette is usually small in size (usually smaller and more discreet than vape pens), easily portable, and easy to use.

A common vaporizer comprises a container, which may be a tank-which is typically refillable, or a cartridge-which is typically single-use and not refillable. The tank or cartridge holds a liquid often referred to as an e-liquid or e-juice. Tanks typically are made out of polycarbonate plastic, glass, or stainless steel. The vaporizer also includes a mouthpiece for inhaling by a person through the mouth; an atomizer comprising a tiny heating element that converts the liquid into tiny, airborne droplets that are inhaled; and a controller for turning on the atomizer. Many vape pens are mouth-activated and turn on automatically when a person inhales. Others vape pins are button activated and require the person to push a button to activate the atomizer. Vaporizers are electrically powered using one or more batteries. The batteries typically are lithium ion batteries that are rechargeable and primarily are used to heat the heating element of the atomizer. A charger usually accompanies a vaporizer when purchased for charging the batteries. The charger may be a USB charger, car charger, or wall charger, and such chargers are generally similar to phone chargers.

The battery-powered vaporizer produces vapor from any of a variety of liquids and liquid mixtures, especially those containing nicotine or cannabinoids. Many diverse types and flavors are available. Moreover, the liquids can be non-medicated (i.e., containing no nicotine or other substances-just pure vegetable glycerin and flavoring), or the liquids can contain nicotine or even in some instances if and where legal, the liquids can contain THC/CBD. The liquids also may contain one or more of a variety of flavors as well as micronutrients such as, for example, vitamin B12. A person can mix the liquids for use with a vape pen. Vaporizers typically are purchased with prefilled cartridges. The heating element turns the contents of the liquids into an aerosol-the vapor-that is inhaled into the lungs and then exhaled by the person. Perhaps one of the most popular vaporizers today is known as the "JUUL", which is a small, sleek device that resembles a computer USB flash drive.

It is believed that, while promoted as healthier than traditional cigarette use, vaping actually may be more dangerous. Propylene glycol, vegetable glycerin and combinations or methylations thereof, are chemicals that are often mixed with nicotine, cannabis, or hemp oil for use in vaporizers. Propylene glycol is the primary ingredient in a majority of nicotine-infused e-cigarette liquids. Unfortunately, at high temperatures propylene glycol converts into tiny polymers that can wreak havoc on lung tissue. In particular, scientists know a great deal about propylene glycol. It is found in a plethora of common household items-cosmetics, baby wipes, pharmaceuticals, pet food, antifreeze, etc. The U.S. Food and Drug Administration and Health Canada have deemed propylene glycol safe for human ingestion and topical application. But exposure by inhalation is another matter. Many things are safe to eat but dangerous to breathe. Because of low oral toxicity, propylene glycol is classified by the FDA as "generally recognized as safe" (GRAS) for use as a food additive, but this assessment was based on toxicity studies that did not involve heating and breathing propylene glycol. Indeed, a 2010 study published in the International Journal of Environmental Research and Public Health concluded that airborne propylene glycol circulating indoors can induce or exacerbate asthma, eczema, and many allergic symptoms. Children were said to be particularly sensitive to these airborne toxins. An earlier toxicology review warned that propylene glycol, ubiquitous in hairsprays, could be harmful because aerosol particles lodge deep in the lungs and are not respirable.

Moreover, when propylene glycol is heated, whether by a red-hot metal coil of a heating element of a vaporizer or otherwise, the potential harm from inhalation exposure increases. It is believed that high voltage heat transforms the propylene glycol and other vaping additives into carbonyls. Carbonyls are a group of cancer-causing chemicals that includes formaldehyde, which has been linked to spontaneous abortions and low birth weight. A known thermal breakdown product of propylene glycol, formaldehyde is an International Agency for Research on Cancer group 1 carcinogen!

Prevalent in nicotine e-cigarette products and present in some vape oil cartridges, FDA-approved flavoring agents pose additional risks when inhaled rather than eaten. The flavoring compounds smooth and creamy (diacetyl and acetyl propionyl) are associated with respiratory illness when inhaled in tobacco e-cigarette devices. Another hazardous-when-inhaled-but-safe-to-eat flavoring compound is Ceylon cinnamon, which becomes cytotoxic when aerosolized.

When a heating element gets red hot in a vaporizer, the liquid undergoes a process called "smoldering", which is a technical term for what is tantamount to "burning"; while much of the liquid is vaporized and atomized, a portion of the liquid undergoes pyrolysis or combustion. In that sense, most of the vaporizers that have flooded the commercial market may not be true vaporizers.

It thus will be appreciated that as inhalation delivery systems using heating have increased in prominence, concerns about their short and long term safety have come into focus. This is particularly true for vaping where there exist ongoing concerns about the possible presence of harmful and potentially harmful constituents (HPHCs) in the inhaled vapor.

Additionally, clearance mechanisms of the lung, like all major points of contact with the external environment, have evolved to prevent the invasion of unwanted airborne particles from entering the body. Airway geometry, humidity and clearance mechanisms contribute to this filtration process. Inhalation delivery systems are often unable to provide the desired effect to a user because the pre-vaporized liquid becomes unstable over time or the active ingredient itself is not properly sized or dispersed for deposition in the alveolar lung. This is a problem not only for vaping, but for other inhalation delivery systems that play an increasing role in the targeted delivery of active ingredients to the human pulmonary system. This is true both for medical purposes, such as the targeted delivery of anticancer medications to the lungs, as well as for recreational/personal purposes, such as vaping.

In view of the foregoing, it is believed that a need exists for a vaporizer that provides an aerosol of the desired chemicals without the harmful byproducts that arise from smoldering. It is also believed that a need exists for a vaporizer that effectively and efficiently produces a vapor cloud that is not inhibited by the body's natural filtration process. It further is believed that a need exists for an active ingredient delivery system that enhances the shelf-life of the pre-vaporized liquid component and enhances the efficacy of the desired treatment/effect, while avoiding the presence of undesired HPHCs in the inhaled vapor. One or more of these needs, and still other needs, are believed to be met by one or more respective embodiments in accordance with one or more aspects and features of the invention.

WO2019239217A1 discloses a mobile inhaler configured to provide an inhalable aerosol to a user. The mobile inhaler may include a vibrating mesh atomizer. The vibrating mesh atomizer may include a vibrating mesh membrane comprising a plurality of holes, a piezoelectric actuator coupled to the vibrating mesh membrane, wherein the processing equipment is configured to actuate the piezoelectric actuator, and a vibrating mesh membrane holder. The mobile inhaler includes processing equipment, which may include memory configured to store information about one or more users, including usage behaviour. A capsule is used to hold liquid for atomizing and may include a plunger to dispense liquid. The processing equipment may be configured to determine desired dosages, or other desired usage metrics, based on the stored information. In some embodiments, information from a plurality of mobile inhalers may be stored and analysed.

US20030192959A1 discloses an apparatus for freshening air, comprising: a base unit having a recess for engaging at least one cartridge; a power supply operably connected to the base unit; an active portion of a nebulizer that includes a piezoelectric element connected to be driven by a driving and switching circuit connected to the power supply, wherein the active portion is incorporated with the base unit; a detachable autonomous liquid droplet dispensing cartridge detachably engagable with the recess of the base unit, wherein the cartridge comprises a first airless bag for storing a first nebulizable liquid, a second airless bag for storing a second nebulizable liquid, a passive portion of the nebulizer, wherein the passive portion includes an interface; a casing enclosing the first and second bags, and housing the passive portion; wherein when the dispensing cartridge engages the recess of the base unit, the passive portion is connected to the active portion.

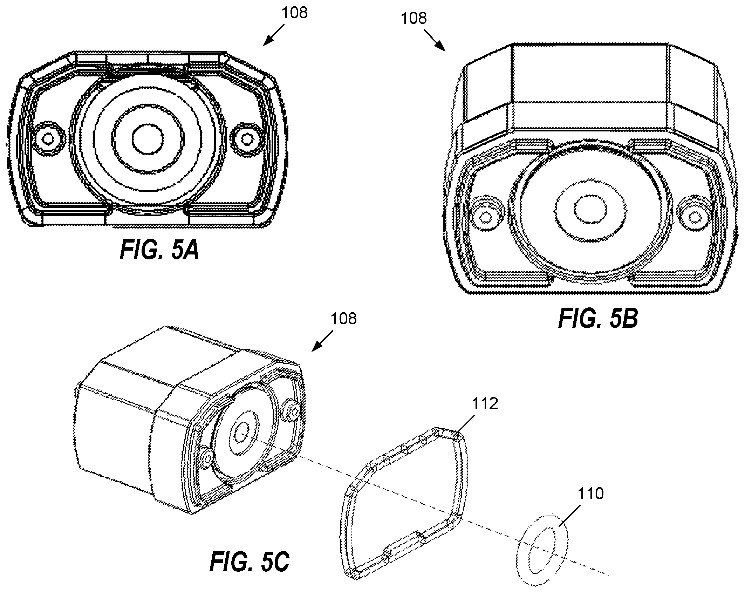

US5894841A discloses a reservoir (10) of a physiologically active substance and a droplet ejection device (14), for example a bubble jet or pizeoelectric device, which is controlled to issue a predetermined number of discrete droplets of the substance from ejection orifices (15) upon actuation. Device (14) may be actuated by a pressure transducer (19) responsive to inhalation and issue the droplets into an airstream (A) which enters at slot (7) and is then inhaled via mouthpiece (5). In other embodiments (FIG. 5) the dispenser is finger actuated and directed by hand for topical application. The number and/or frequency of droplets issued is programmatically controlled by a control circuit (16) whereby average and total dose of the substance are predetermined.

US20160228658A1 discloses a thermally modulating inhalable medicament delivery device may deliver an inhalable medicament as an aerosol, vapor, or partial aerosol and partial vapor mixture. The inhalable medicament may be delivered to a target in a subject. Described herein are devices, systems, and methods for delivering an inhalable medicament to a subject.

US10575564B2 discloses an aerosol delivery device or electronic cigarette which generates an aerosol or vapor for consumption by a consumer. The device may include a cartridge holding an aerosol precursor substance or fluid that is turned into the aerosol or vapor. The fluid may be stored in reservoir that allows for the fluid to be passed to an atomizer for generating the aerosol. The reservoir may be a flexible bladder that equalizes pressure inside the cartridge to reduce leakage, such as with an internal valve to activate fluid transfer. Alternatively, the reservoir may include one or more capsules that can be broken or melted to release the fluid.

WO2020234807A1 discloses an aerosol delivery device that may comprise a housing defining an outer wall. The device may further include a power source and a control component, a mouthpiece portion, a tank portion that includes a reservoir configured to contain a liquid composition, and an atomization assembly configured to vaporize the liquid composition to generate an aerosol. The atomization assembly may comprise a vibrating assembly that includes a mesh plate. In some implementations, the reservoir of the aerosol delivery device may be configured to rotate relative to the position of the aerosol delivery device. In some implementations, the aerosol delivery device may further comprise a perforated gate. In some implementations, the aerosol delivery device may further comprise a liquid transport element. In some implementations, the aerosol deliver device may further comprise a micropump assembly. In some implementations, the reservoir of the aerosol delivery device may be U-shaped.

### SUMMARY OF THE INVENTION

The invention is defined in the appended claims. The disclosure includes many aspects and features, and generally relates to apparatus, systems, formulations, and methods pertaining to liquids that are aerosolized and inhaled by persons using electronic devices, whether intended for personal or recreational use, or for the administration of medicines. Moreover, while many aspects and features relate to, and are described in, the context of vaping, the invention is not limited to use only in such context. Indeed, depending on the context of use, the electronic device of the invention may be considered a vaporizer and may be in the form of a vape pen or e-cigarette. Indeed, those who vape may come to refer to embodiments of the invention as a vape pen even though heat is not utilized to create the aerosol that is inhaled. In the delivery of pharmaceuticals, patients may come to refer to embodiments of the invention as a nebulizer even though a gas transport (e.g., compressed gas) is not utilized and even though the aerosol that is produced in accordance with the invention may have a smaller particle size than the mist produced by common nebulizers. Other separate and distinct contexts of use of embodiments of the invention may similarly result in different nomenclature of the embodiments of the invention. Nonetheless, while the appearance and form factor of embodiments of the invention may vary depending on such contexts of use, the basic components and operation remain the same, except where otherwise described below.

In an aspect of the invention, a cartridge assembly is configured to couple with a handheld base assembly and, preferably, are configured to magnetically couple. The handheld base assembly comprises electronics in the form of a printed circuit board and a power source in the form of a battery, which preferably is rechargeable. An electrical connection is made when the cartridge assembly is coupled with the handheld base assembly by which the cartridge assembly is powered. The base also preferably includes magnets that magnetically attract a metal plate of the cartridge assembly to secure the cartridge assembly within an opening in an end of the base. The cartridge assembly preferably is a disposable cartridge assembly.

In accordance with this aspect, the cartridge assembly comprises a cartridge and a bladder assembly contained within the cartridge. In turn, the bladder assembly comprises a bladder containing a wick and a mesh assembly that sits on top of and covers a mouth of the bladder. The wick acts to draw liquid to the mesh assembly. The wick is retained in physical engagement with the bladder proximate its bottom by protuberances that extend from the walls of the bladder. There are preferably four protuberances that surround the end of the wick in a discontinuous circular pattern and receive the end of the wick in frictional fit with each of four sides. The wick extends therefrom to and is retained in physical engagement with the mesh assembly and, in particular, a piezoelectric disk having a mesh material which, when powered by the power source, vibrates so as to aerosolize a liquid contained within the bladder and wick. The mesh assembly is held in tension on top of a lip of the mouth of the bladder by a sealing O-ring that is forced into engagement with the mesh assembly by the attachment of a mouthpiece of the cartridge assembly to the cartridge. Screws are preferably utilized in effecting the attachment whereby the force by which the O-ring is held in contact with the mesh assembly may be adjusted. A spacer on a printed circuit board of the cartridge assembly may additionally engage the bottom of the silicone bladder and hold the wick in tension therethrough. Due to these features, it is believed that the bladder and wick ensure that the mesh remains in constant contact with the liquid for consistent aerosolization each time the electronic device is triggered. The liquid preferably is supplied to the vibrating mesh at a generally constant pressure whereby a generally uniform aerosol is produced, and this is accomplished regardless of the orientation of the electronic device.

In a feature, the cartridge assembly comprises a printed circuit board or other electronics, and the cartridge assembly communicates with the handheld base assembly when coupled. Preferably, the printed circuit board includes memory that includes information regarding the liquid contained in the bladder and dosing information related thereto, e.g., the number of doses dispensed so far from the cartridge assembly. The cartridge assembly further can be programmed to only work with one or more specified handheld base assemblies to the exclusion of other handheld base assemblies. For example, a cartridge assembly could be configured to work only with a handheld base assembly of a particular person, e.g., a certain patient for whom a prescription is provided via the cartridge assembly.

In a feature, the wick has a lengthwise channel that extends between its opposite ends. The channel assists in delivery liquid to the mesh assembly for aerosolizing.

In a feature, the wick is rigid.

In a feature, opening cross sections of the mesh that is in contact with the liquid is smaller than the opening cross section that faces the mouthpiece and exit of the aerosolized liquid. The taper angle and size of the perforated mesh preferably is adjusted via electro-forming methods to achieve a laminar and non-turbulent aerosol that is best suited for deep lung penetration and will, therefore, not yield large amounts of buccal deposition.

In a feature, an airflow channel is defined between the port of the mouthpiece and a pressure sensor located within the handheld base assembly. A D-ring is provided to seal the interface between the cartridge and the mouthpiece to prevent loss of suction along the airflow channel. The airflow channel is defined by openings in the mouthpiece, the cartridge, the printed circuit board, the metal plate, and the chassis. Furthermore, while one opening is shown in connection with the mouthpiece in the drawings, three openings preferable are provided that are equally spaced around the O-ring.

In an aspect, the bladder may be filled with the liquid by injection after assembly of the cartridge assembly. Since the bladder preferably is a self-sealing silicone bladder, when the injector needle is removed, the bladder re-seals and no liquid drains or leaks out. In this aspect, the liquid may be injected as a last stop via an access port/injector port that is located on the bottom of the cartridge. Alternatively, the bladder is inserted into the cartridge and then is filled with liquid first (top-down pour) without utilizing a needle or puncturing the bladder with an injector needle. In this manner, the bladder is filled by pouring liquid into the bladder and, once the desired volume has been dispensed, the wick is inserted inside the bladder and then the bladder is capped off by the mesh assembly and the rest of the cartridge assembly is then assembled. The cartridge assembly preferably is a disposable cartridge assembly.

Additional aspects and features of a preferred commercial system and apparatus for dosing by patients are disclosed in the drawings.

In addition to the aforementioned aspects and features of the invention, it should be noted that the invention further encompasses the various logical combinations and subcombinations of such aspects and features. Thus, for example, claims in this or a divisional or continuing patent application or applications may be separately directed to any aspect, feature, or embodiment disclosed herein, or combination thereof, without requiring any other aspect, feature, or embodiment.

### BRIEF DESCRIPTION OF THE DRAWINGS

One or more preferred embodiments of the invention now will be described in detail with reference to the accompanying drawings, wherein the same elements are referred to with the same reference numerals.
FIG. 1A illustrates an electronic device having a handheld base assembly and a cartridge assembly, in accordance with one or more preferred implementations of the invention.
FIG. 1B illustrates the act of separating the handheld base assembly from the cartridge assembly in the electronic device of FIG. 1A.
FIG. 1C illustrates a close-up view of the cartridge assembly after being separated from the handheld base assembly as illustrated in FIG. 1B.
FIG. 2A illustrates a front elevational view of the cartridge assembly of FIG. 1C.
FIG. 2B illustrates a rear elevational view of the cartridge assembly of FIG. 1C.
FIG. 2C illustrates a perspective view of the rear and a bottom of the cartridge assembly of FIG. 1C.
FIG. 2D illustrates a perspective view of the bottom of the cartridge assembly of FIG. 1C.
FIG. 2E illustrates a perspective view of the bottom and a first side of the cartridge assembly of FIG. 1C.
FIG. 2F illustrates a perspective view of the first side of the cartridge assembly of FIG. 1C.
FIG. 3A illustrates a perspective view of the first side and a top of the cartridge assembly of FIG. 1C.
FIG. 3B illustrates a perspective view of the top of the cartridge of FIG. 1C.
FIG. 3C illustrates a perspective view of the top and a second side of the cartridge assembly of FIG. 1C.
FIG. 3D illustrates a perspective view of the second side of the cartridge assembly of FIG. 1C.
FIG. 3E illustrates a perspective view of the second side and the bottom of the cartridge assembly of FIG. 1C.
FIG. 3F illustrates a perspective view of the front, first side, and top of the cartridge assembly of FIG. 1C.
FIG. 4A is an exploded view of the cartridge assembly of FIG. 1C.
FIG. 4B is a perspective view of a top and a rear of a mouthpiece of the cartridge assembly of FIG. 4A.
FIG. 4C is an elevational view of the rear of the mouthpiece of FIG. 4B.
FIG. 5A is an elevational view of a front of the cartridge of the cartridge assembly of FIG. 1C.
FIG. 5B is a perspective view of the front and a top of the cartridge of FIG. 1C.
FIG. 5C is an exploded perspective view of the front, top, and a first side of the cartridge of FIG. 1C, wherein an O-ring and D-ring are removed from the remainder of the cartridge.
FIG. 6A is a perspective view of the top and front of the cartridge of FIG. 1C, wherein the O-ring and D-ring have been removed from remainder of the cartridge.
FIG. 6B is an exploded perspective view of the top, front, and the first side the cartridge of FIG. 1C, wherein two fasteners and a metal plate are removed from the remainder of the cartridge.
FIG. 6C is a perspective view of the top, side, and a rear of the cartridge of FIG. 6B, wherein the two fasteners and metal plate have been removed from the remainder of the cartridge.
FIG. 7A is an exploded perspective view of the top, rear, and first side of the cartridge of FIG. 6C, wherein a circuit board is removed from the remainder of the cartridge.
FIG. 7B is an exploded perspective view of the top, rear, and first side of the cartridge of FIG. 7A, wherein a bladder assembly of the cartridge is removed from the remainder of the cartridge.
FIG. 8A is a perspective view of a front of the remainder of the cartridge of FIG. 7B after the bladder assembly has been removed.
FIG. 8B is a perspective view of the front and a first side of the remainder of the cartridge of FIG. 8A.
FIG. 8C is a perspective view of a rear of the remainder of the cartridge of FIG. 8A.
FIG. 9 is an exploded perspective view of the bladder assembly of FIG. 7B, wherein the cartridge bladder assembly including a bladder, a wick, and a mesh assembly.
FIG. 10A is a perspective view of a top of the bladder of FIG. 9.
FIG. 10B is a perspective view of the top and a front of the bladder of FIG. 9.
FIG. 10C is a perspective view of the front of the bladder of FIG. 9.
FIG. 10D is a perspective view of a first side the bladder of FIG. 9.
FIG. 10E is a perspective view of the top and the first side the bladder of FIG. 9.
FIG. 11A is the view of FIG. 10A illustrated only with shading.
FIG. 11B is the view of FIG. 10B illustrated only with shading.
FIG. 11C is the view of FIG. 10C illustrated only with shading.
FIG. 11D is the view of FIG. 10D illustrated only with shading.
FIG. 11E is the view of FIG. 10E illustrated only with shading.
FIG. 12A is a perspective view illustrated only with shading of the bladder of FIG. 9.
FIG. 12B is a perspective view illustrated only with shading of the bladder and the wick of FIG. 9.
FIG. 13A is a top plan view of the wick of FIG. 9.
FIG. 13B is a side elevational view of the wick of FIG. 9, wherein a longitudinal channel of the wick is shown.
FIG. 13C is a perspective view of the top and the side of the wick of FIG. 9, wherein the longitudinal channel of the wick is shown.
FIG. 13D is a perspective view of a bottom and the side of the wick of FIG. 9, wherein the longitudinal channel of the wick is shown.
FIG. 14A is a top plan view of the mesh assembly of FIG. 9, wherein electrical connections are omitted for clarity.
FIG. 14B is a perspective view of the top and a side of the mesh assembly of FIG. 14A.
FIG. 14C is a perspective view of the side and a bottom of the mesh assembly of FIG. 14A.
FIG. 14D is a bottom plan view of the mesh assembly of FIG. 14A.
FIG. 15 is a perspective view of the handheld base assembly of FIG. 1B.
FIG. 16 is the perspective view of the handheld base assembly of FIG. 15, wherein the skin and tail are omitted.
FIG. 17A is a front elevational view of the handheld base assembly of FIG. 16.
FIG. 17B is a rear elevational view of the handheld base assembly of FIG. 16.
FIG. 17C is a top plan view of the handheld base assembly of FIG. 16.
FIG. 17D is an elevational view of a first side of the handheld base assembly of FIG. 16.
FIG. 17E is a bottom plan view of the handheld base assembly of FIG. 16.
FIG. 17F is an elevational view of a second, opposite side of the handheld base assembly of FIG. 16.
FIG. 18 is the perspective view of FIG. 16, wherein the chassis is further omitted.
FIG. 19A is a partial, cross-sectional view of a distal end of the front of the handheld base assembly of FIG. 16.
FIG. 19B is another partial, cross-sectional view of the distal end of the front of the handheld base assembly of FIG. 16.
FIG. 19C is a partial view of the distal end of the front of the handheld base assembly of FIG. 16 similar to that of FIG. 19 B, but not in cross-section.
FIG. 20A is a perspective view of the metal plate of FIG. 6B.
FIG. 20B is a perspective view of the printed circuit board of FIG. 7A.
FIG. 20C is a front elevational view of the cartridge and printed circuit board, wherein the cartridge is transparently shown.
FIG. 21 is a view of disassembled components of a cartridge assembly of a prototype commercial embodiment of the disclosure.
FIG. 22 is a perspective view of the bladder of the cartridge assembly of FIG. 21.
FIG. 23 is a perspective view of the bladder of FIG. 22 positioned within the cartridge of FIG. 21.
FIG. 24A is an elevational view of a top of a prototype commercial embodiment of the disclosure.
FIG. 24B is an elevational view of a top of a prototype commercial embodiment of the disclosure, wherein "WAVE" is being displayed on a display.
FIG. 24C is an elevational view of the top of the prototype commercial embodiment of the disclosure, wherein a dose count of 97 is being displayed on the display.
FIG. 24D is a perspective partial view of the prototype commercial embodiment while a vapor is emitted from the mouthpiece.
FIG. 24E is a perspective view of the prototype commercial embodiment as an upward force is applied to the cartridge assembly using two fingers while the base assembly is held by the remaining fingers of a hand.
FIG. 24F is a perspective view of the prototype commercial embodiment following the application of the upward force FIG. 24E, wherein the cartridge assembly has been decoupled and removed from the handheld base assembly.
FIG. 24G is a view of the prototype commercial embodiment wherein the decoupled cartridge assembly has been inverted to show a bottom thereof.
FIG. 25 is a perspective view of another prototype commercial embodiment of a preferred commercial system and apparatus for dosing by patients.

### DETAILED DESCRIPTION

As a preliminary matter, it will readily be understood by one having ordinary skill in the relevant art ("Ordinary Artisan") that the invention has broad utility and application. Furthermore, any embodiment discussed and identified as being "preferred" is considered to be part of a best mode contemplated for carrying out the disclosure. Other embodiments also may be discussed for additional illustrative purposes in providing a full and enabling disclosure.

Accordingly, while the invention is described herein in detail in relation to one or more embodiments, it is to be understood that this disclosure is illustrative and exemplary of the invention and is made merely for the purposes of providing a full and enabling disclosure of the invention. The detailed disclosure herein of one or more embodiments is not intended, nor is to be construed, to limit the scope of patent protection afforded the invention in any claim of a patent issuing here from, which scope is to be defined by the claims and the equivalents thereof. It is not intended that the scope of patent protection afforded the invention be defined by reading into any claim a limitation found herein that does not explicitly appear in the claim itself.

Thus, for example, any sequence(s) and/or temporal order of steps of various processes or methods that are described herein are illustrative and not restrictive. Accordingly, it should be understood that, although steps of various processes or methods may be shown and described as being in a sequence or temporal order, the steps of any such processes or methods are not limited to being carried out in any particular sequence or order, absent an indication otherwise. Indeed, the steps in such processes or methods generally may be carried out in various different sequences and orders while still falling within the scope of the disclosure.

Additionally, it is important to note that each term used herein refers to that which the Ordinary Artisan would understand such term to mean based on the contextual use of such term herein. To the extent that the meaning of a term used herein-as understood by the Ordinary Artisan based on the contextual use of such term-differs in any way from any particular dictionary definition of such term, it is intended that the meaning of the term as understood by the Ordinary Artisan should prevail.

With regard to the construction of the scope of any claim in the United States, no claim element is to be interpreted under 35 U.S.C. 112(f) unless the explicit phrase "means for" or "step for" is actually used in such claim element, whereupon this statutory provision is intended to and should apply in the interpretation of such claim element. With regard to any method claim including a condition precedent step, such method requires the condition precedent to be met and the step to be performed at least once but not necessarily every time during performance of the claimed method.

Furthermore, it is important to note that, as used herein, "comprising" is open-ended insofar as that which follows such term is not exclusive. Additionally, "a" and "an" each generally denotes "at least one" but does not exclude a plurality unless the contextual use dictates otherwise. Thus, reference to "a picnic basket having an apple" is the same as "a picnic basket comprising an apple" and "a picnic basket including an apple", each of which identically describes "a picnic basket having at least one apple" as well as "a picnic basket having apples"; the picnic basket further may contain one or more other items beside an apple. In contrast, reference to "a picnic basket having a single apple" describes "a picnic basket having only one apple"; the picnic basket further may contain one or more other items beside an apple. In contrast, "a picnic basket consisting of an apple" has only a single item contained therein, i.e., one apple; the picnic basket contains no other item.

When used herein to join a list of items, "or" denotes "at least one of the items" but does not exclude a plurality of items of the list. Thus, reference to "a picnic basket having cheese or crackers" describes "a picnic basket having cheese without crackers", "a picnic basket having crackers without cheese", and "a picnic basket having both cheese and crackers"; the picnic basket further may contain one or more other items beside cheese and crackers.

When used herein to join a list of items, "and" denotes "all of the items of the list". Thus, reference to "a picnic basket having cheese and crackers" describes "a picnic basket having cheese, wherein the picnic basket further has crackers", as well as describes "a picnic basket having crackers, wherein the picnic basket further has cheese"; the picnic basket further may contain one or more other items beside cheese and crackers.

The phrase "at least one" followed by a list of items joined by "and" denotes an item of the list but does not require every item of the list. Thus, "at least one of an apple and an orange" encompasses the following mutually exclusive scenarios: there is an apple but no orange; there is an orange but no apple; and there is both an apple and an orange. In these scenarios if there is an apple, there may be more than one apple, and if there is an orange, there may be more than one orange. Moreover, the phrase "one or more" followed by a list of items joined by "and" is the equivalent of "at least one" followed by the list of items joined by "and".

Referring now to the drawings, one or more preferred embodiments in accordance with one or more aspects and features of the disclosure are next described. The following description of one or more preferred embodiments is merely exemplary in nature and is in no way intended to limit the disclosure, its implementations, or uses.

In accordance with electronic devices of the disclosure, a vibrating mesh is provided for aerosolizing a liquid without smoldering. The aerosolized liquid preferably is in the form of a vapor cloud similar to what a person or observer would surmise to be "vapor" when vaping. In the context of vaping, such preferred devices of the disclosure therefore are believed to produce an aerosol that is free of undesired carcinogens. This is in stark contrast to vaporizers used today to aerosolize e-liquids by heating the e-liquids and desired compounds contained therein ( e.g., nicotine) or supplements such as B12, THC/CBD and other drugs or stimulants. As a result of using heating to aerosolize the e-liquids, these vaporizers produce toxic byproducts like formaldehyde, a recognized Group 1 carcinogen for cancer, which toxic byproducts then are unfortunately inhaled by a person using the vaporizer. For example, when the liquids are heated, the liquids undergo a thermochemical reaction producing unwanted emissions. The unwanted emissions of the toxic byproducts may cause bodily harm from extended inhalation exposure.

By utilizing a vibrating mesh, preferred electronic devices in accordance with one or more aspects and features of the disclosure produce an aerosol without using heat and thus advantageously avoid such toxic byproducts created by the vaporizes currently on the market. The electronic devices thereby advantageously produce a carcinogen free aerosol free of harmful emission byproducts.

The preferred electronic devices in accordance with one or more aspects and features of the disclosure are particularly well suited for aerosolizing a liquid for inhalation without heating and, in particular, for aerosolizing an aqueous formulation including nicotine for inhalation without heating.

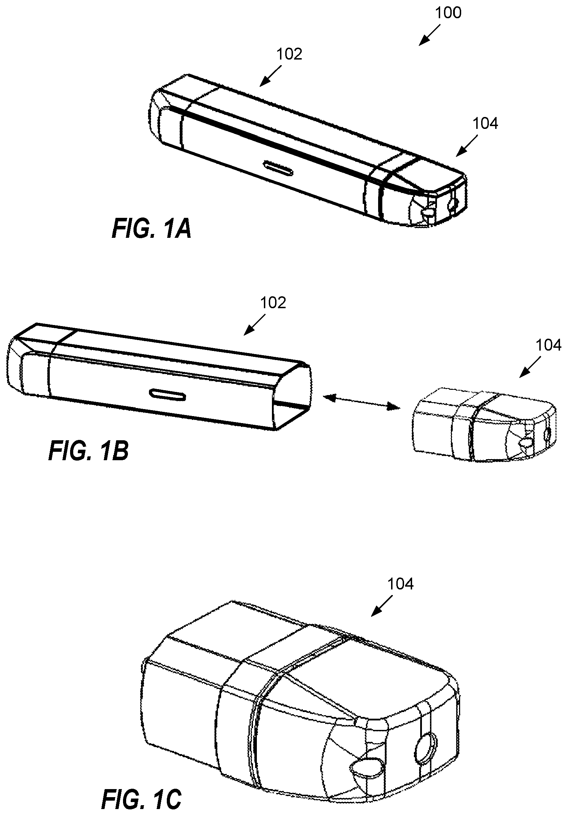

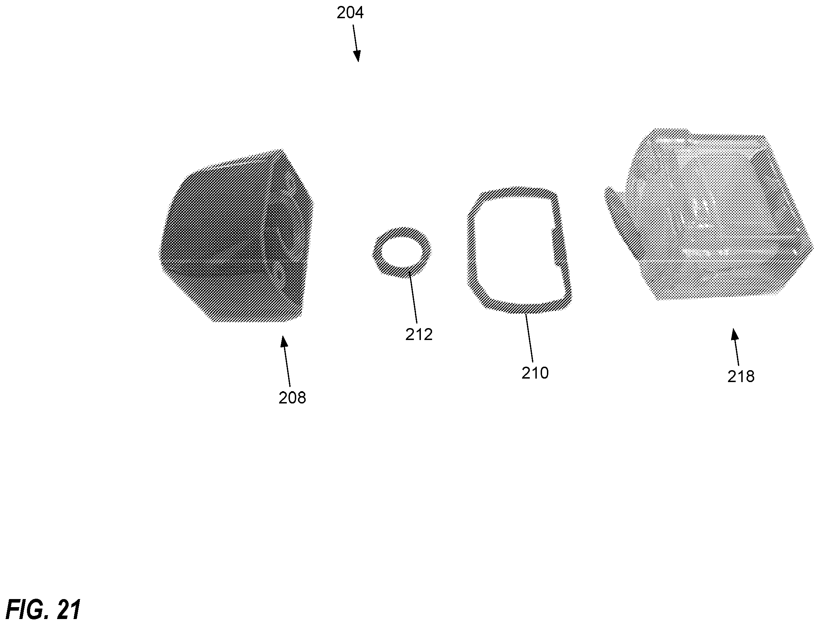

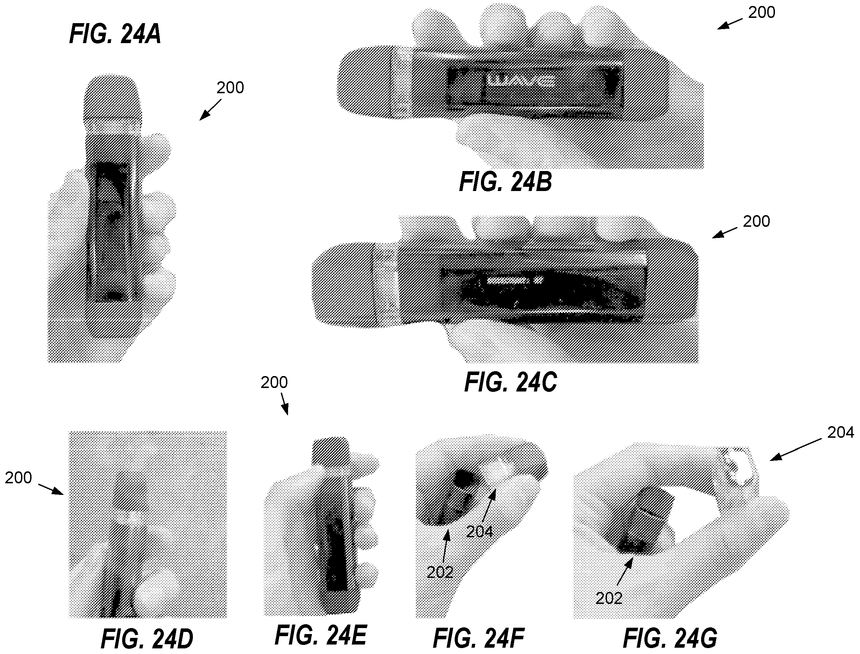

Such a preferred embodiment of an electronic device is illustrated in and described with reference to FIG. 1A through FIG. 20C in accordance with one or more aspect and features of the disclosure. Components for a prototype cartridge assembly are seen in FIGS. 21, 22, and 23. A commercial prototype is seen in FIGS. 24A through 24G.

Other forms of an electronic device in accordance with the present disclosure include vapes, vape pens, and nebulizers. Other terminology may be given to electronic devices of the present disclosure. In any event, electronic devices of the present invention produce an aerosol for inhalation without smoldering or heating, whatever commercial or consumer name may be given.

FIG. 1A illustrates an electronic device **100** having a handheld base assembly **102** and a cartridge assembly **104,** in accordance with one or more preferred implementations of the invention. The cartridge assembly and handheld base assembly are configured to removably couple together. FIG. 1B illustrates the act of separating the handheld base assembly **102** from the cartridge assembly **104** in the electronic device of FIG. 1A. Preferably, the cartridge assembly magnetically mounts onto an end of the handheld base assembly for magnetic, decoupling attachment. FIG. 1C illustrates a close-up view of the cartridge assembly **104** after being separated from the handheld base assembly as illustrated in FIG. 1B.

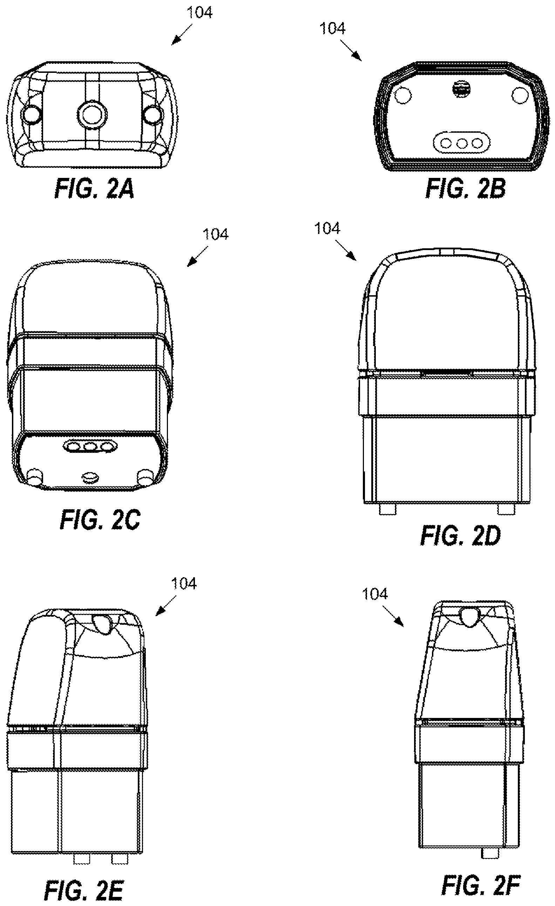

With regard to FIGS. 2A-2F, FIG. 2A illustrates a front elevational view of the cartridge assembly **104** of FIG. 1C; FIG. 2B illustrates a rear elevational view of the cartridge assembly **104** of FIG. 1C; FIG. 2C illustrates a perspective view of the rear and a bottom of the cartridge assembly **104** of FIG. 1C; FIG. 2D illustrates a perspective view of the bottom of the cartridge assembly **104** of FIG. 1C; FIG. 2E illustrates a perspective view of the bottom and a first side of the cartridge assembly **104** of FIG. 1C; and FIG. 2F illustrates a perspective view of the first side of the cartridge assembly **104** of FIG. 1C.

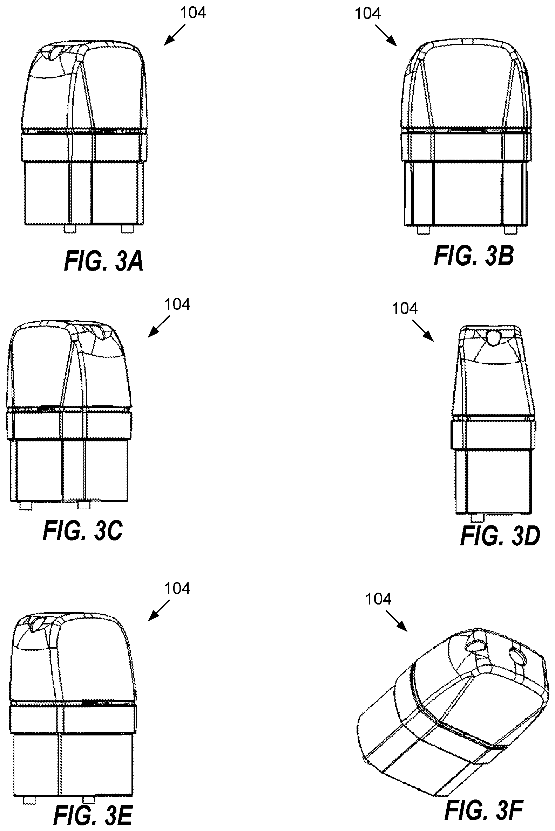

With regard to FIGS. 3A-3F, FIG. 3A illustrates a perspective view of the first side and a top of the cartridge assembly **104** of FIG. 1C; FIG. 3B illustrates a perspective view of the top of the cartridge **104** of FIG. 1C; FIG. 3C illustrates a perspective view of the top and a second side of the cartridge assembly **104** of FIG. 1C; FIG. 3D illustrates a perspective view of the second side of the cartridge assembly **104** of FIG. 1C; FIG. 3E illustrates a perspective view of the second side and the bottom of the cartridge assembly **104** of FIG. 1C; and FIG. 3F illustrates a perspective view of the front, first side, and top of the cartridge assembly **104** of FIG. 1C.

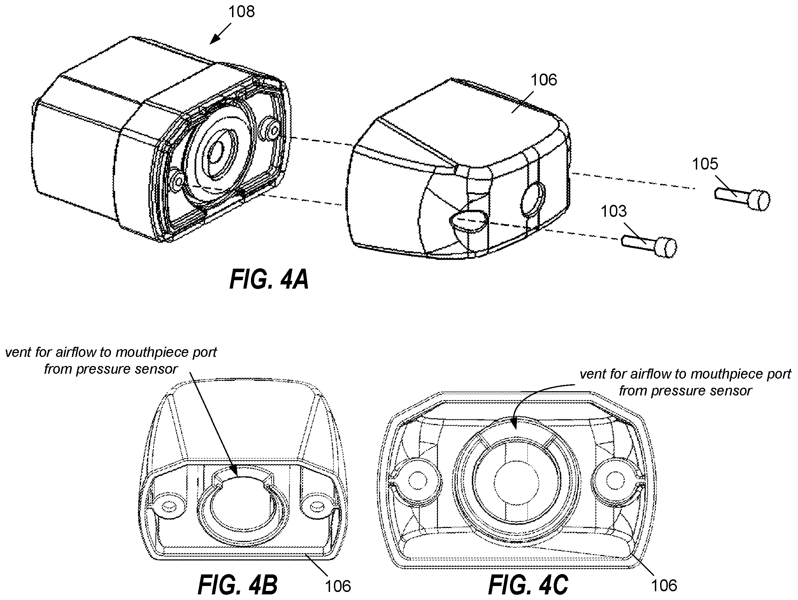

The cartridge assembly **104** comprises a mouthpiece **106;** and a cartridge **108.** FIG. 4A is an exploded view of the cartridge assembly **104** of FIG. 1C, wherein two fasteners **103,105** and the mouthpiece **106** are illustrated being separated from the cartridge **108.** Additionally, FIG. 4B is a perspective view of a top and a rear of a mouthpiece **106** of the cartridge assembly **104** of FIG. 4A; and FIG. 4C is an elevational view of the rear of the mouthpiece **106** of FIG. 4B.

FIG. 5A is an elevational view of a front of the cartridge **108** of the cartridge assembly of FIG. 1C. Additionally, FIG. 5B is a perspective view of the front and a top of the cartridge **108** of FIG. 1C; and FIG. 5C is an exploded perspective view of the front, top, and a first side of the cartridge **108** of FIG. 1C, wherein an O-ring **110** and D-ring **112** are removed from the cartridge **108.** An airflow channel is defined between the port of the mouthpiece and a pressure sensor located within the handheld base assembly, and the D-ring preferably is provided to seal the interface between the cartridge and the mouthpiece to prevent loss of suction along the airflow channel. The airflow channel is defined by openings in the mouthpiece, the cartridge, the printed circuit board, the metal plate, and the chassis. Furthermore, while one opening is shown in connection with the mouthpiece in the drawings, three openings preferable are provided that are equally spaced around the O-ring.

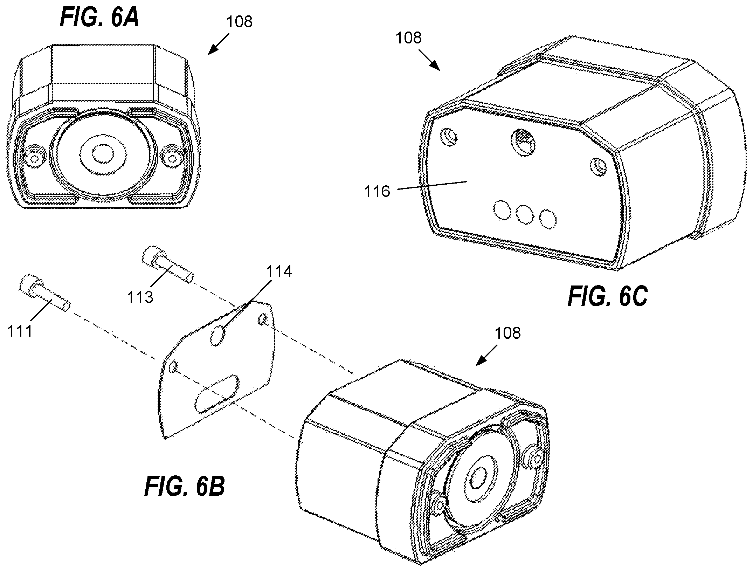

FIG. 6A is a perspective view of the top and front of the cartridge **108** of FIG. 1C, wherein the O-ring **112** and D-ring **110** have been removed from remainder of the cartridge **108.** Additionally, FIG. 6B is an exploded perspective view of the top, front, and the first side the cartridge **108** of FIG. 1C, wherein two fasteners **111,113** and a metal plate **114** are being separated from the remainder of the cartridge **108.** The metal plate **114** is used to couple the cartridge assembly **104** to one or more magnets located in a distal end **140** of the base assembly **102.** FIG. 6C is a perspective view of the top, side, and a rear of the cartridge **108** of FIG. 6B, wherein the two fasteners **111,113** and metal plate **114** have been removed from the remainder of the cartridge **108.**

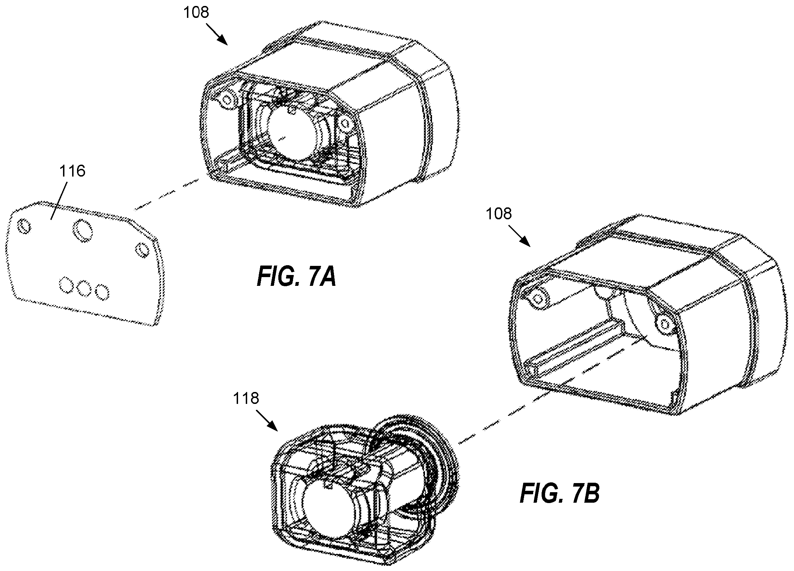

FIG. 7A is an exploded perspective view of the top, rear, and first side of the cartridge **108** of FIG. 6C, wherein a circuit board **116** is removed from the remainder of the cartridge **108.** The cartridge assembly **104** comprises a bladder assembly **118;** and FIG. 7B is an exploded perspective view of the top, rear, and first side of the cartridge **108** of FIG. 7A, wherein a bladder assembly **118** is removed from the cartridge **108.**

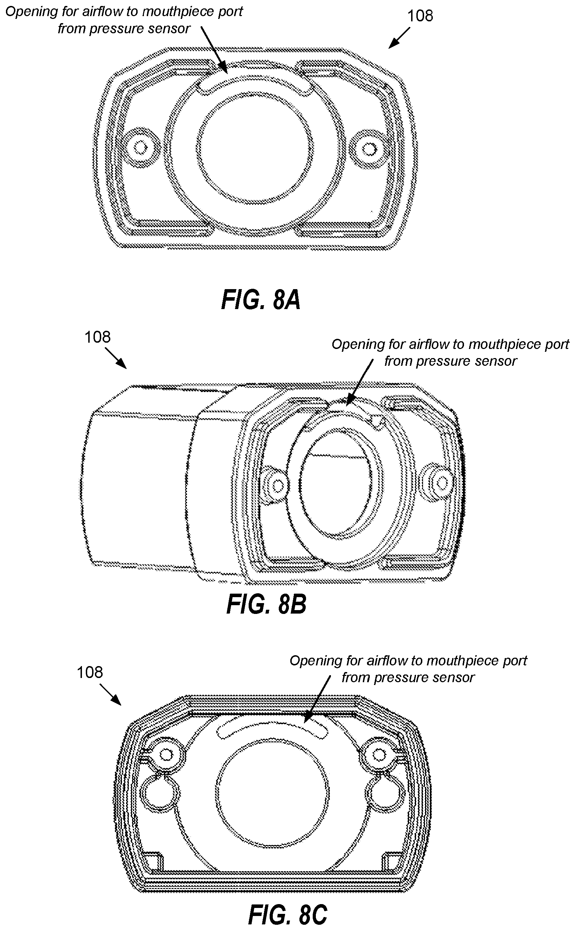

FIG. 8A is a perspective view of a front of the remainder of the cartridge **108** of FIG. 7B after the bladder assembly **118** has been removed. Additionally, FIG. 8B is a perspective view of the front and a first side of the remainder of the cartridge **108** of FIG. 8A; and FIG. 8C is a perspective view of a rear of the remainder of the cartridge **108** of FIG. 8A.

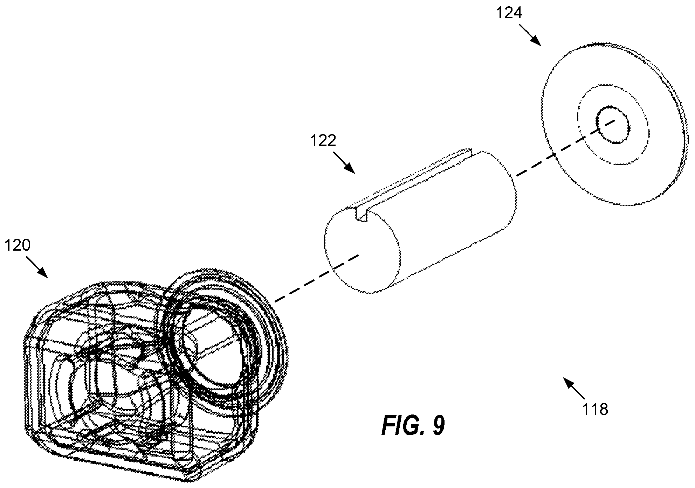

The bladder assembly **118** comprises a bladder **120;** a wick **122** contained within the bladder **120;** and a mesh assembly **124.** FIG. 9 is an exploded perspective view of the bladder assembly **118** of FIG. 7B. The mesh assembly **124** preferably is disposed on top of a lip of a mouth of the bladder **120,** the bladder **120** extending through an opening in the cartridge **108** to define the mouth.

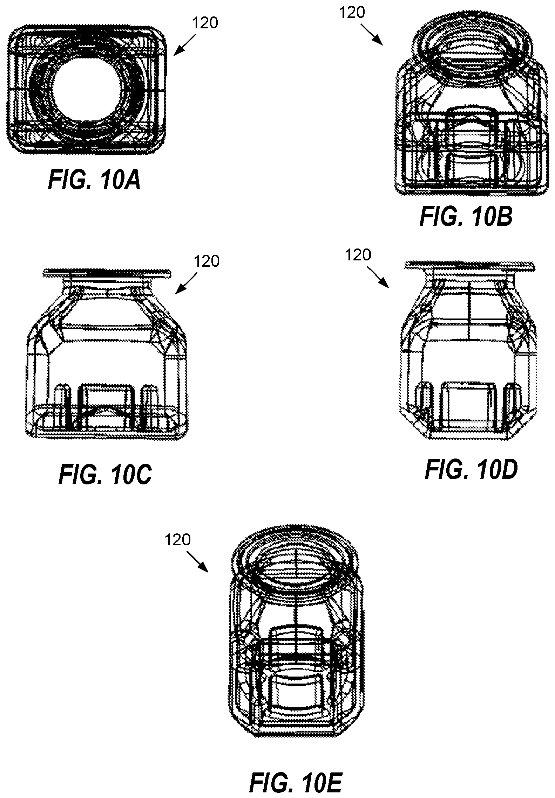

FIG. 10A is a perspective view of a top of the bladder **120** of FIG. 9. Additionally, FIG. 10B is a perspective view of the top and a front of the bladder **120** of FIG. 9; FIG. 10C is a perspective view of the front of the bladder **120** of FIG. 9; FIG. 10D is a perspective view of a first side the bladder **120** of FIG. 9; and FIG. 10E is a perspective view of the top and the first side the bladder **120** of FIG. 9.

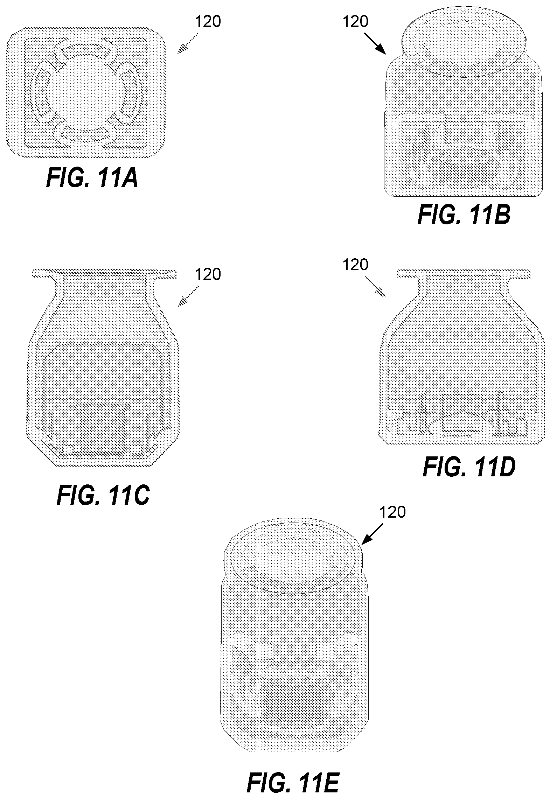

For purposes of further illustration, FIG. 11A is the view of FIG. 10A illustrated only with shading; FIG. 11B is the view of FIG. 10B illustrated only with shading; FIG. 11C is the view of FIG. 10C illustrated only with shading; FIG. 11D is the view of FIG. 10D illustrated only with shading; and FIG. 11E is the view of FIG. 10E illustrated only with shading.

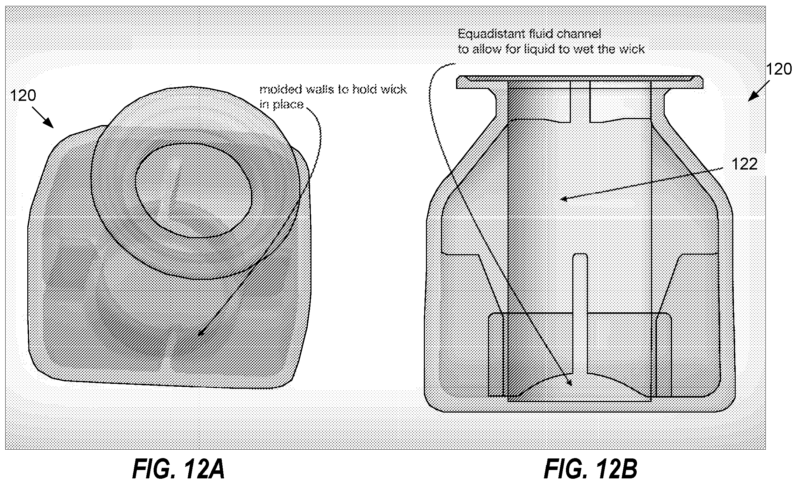

FIG. 12A is a perspective view illustrated only with shading of the bladder **120** of FIG. 9.

FIG. 12B is a perspective view illustrated only with shading of the bladder **120** and the wick **122** of FIG. 9.

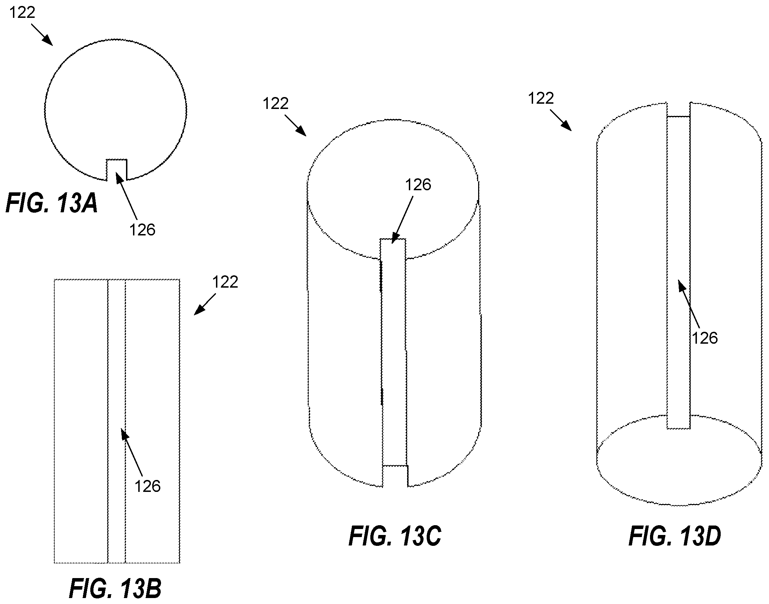

The wick **122** of the bladder assembly **118** preferably comprises a lengthwise channel **126.** FIG. 13A is a top plan view of the wick **122** of FIG. 9; FIG. 13B is a side elevational view of the wick **122** of FIG. 9, wherein a longitudinal channel **126** of the wick **122** is shown; FIG. 13C is a perspective view of the top and the side of the wick **122** of FIG. 9, wherein the longitudinal channel **126** of the wick **122** also is shown; and FIG. 13D is a perspective view of a bottom and the side of the wick **122** of FIG. 9, wherein the longitudinal channel **126** of the wick **122** again is shown. The channel **126** preferably extends between distal ends of the wick **122** and preferably is an open ended channel at both ends, as shown in these figures. The wick may be rigid and the lengthwise channel that extends between its opposite ends assists in delivery liquid to the mesh assembly for aerosolizing.

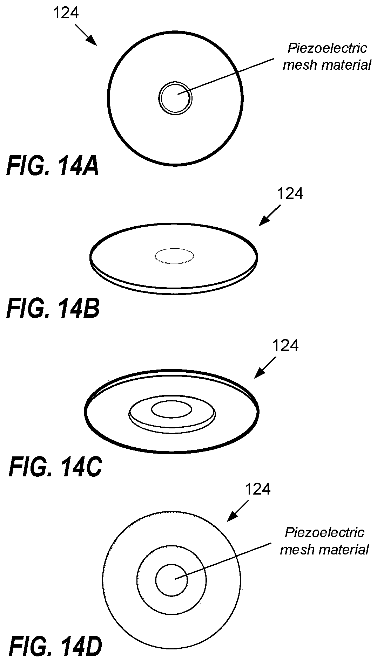

FIG. 14A is a top plan view of the mesh assembly **124** of FIG. 9, wherein electrical connections are omitted for clarity. FIG. 14B is a perspective view of the top and a side of the mesh assembly **124** of FIG. 14A; FIG. 14C is a perspective view of the side and a bottom of the mesh assembly **124** of FIG. 14A; and FIG. 14D is a bottom plan view of the mesh assembly **124** of FIG. 14A.

The mesh assembly **124** comprises a mesh material and a piezoelectric material; preferably, the mesh assembly **124** comprises a piezo mesh disk. Opening cross sections of the mesh that is in contact with the liquid preferably are smaller than the opening cross sections that face the mouthpiece and exit of the aerosolized liquid. The taper angle and size of the perforated mesh preferably is adjusted during manufacture via electro-forming methods so as to achieve a laminar and non-turbulent aerosol that is best suited for deep lung penetration and will, therefore, not yield large amounts of buccal deposition. The mesh material is configured to vibrate when the piezoelectric material is actuated, whereby an aerosol is produced when the mesh material contacts a liquid of the bladder **120** such that the aerosol may be inhaled through the mouthpiece **106.** The wick **122** acts to draw liquid from the bladder **120** to the mesh assembly **124.** The wick **122** preferably is retained in constant physical contact with the mesh assembly **124.** In particular, an end of the wick **122** preferably is secured by the protuberances of the bladder extending from the walls proximate a bottom of the bladder, and the wick has a length such that a distal end of the wick **122** is maintained in contact with the mesh assembly when the bladder assembly **118** is assembled, the opposite end of the wick **122** being held in place by the protuberances of the bladder **120.**

In greater detail, the wick **122** is retained in physical engagement with the bladder **120** proximate its bottom by protuberances that extend from the walls of the bladder. There are preferably four protuberances that surround the end of the wick **122** in a discontinuous circular pattern and receive the end of the wick **122** in frictional fit with each of four sides. The wick **122** extends therefrom to and is retained in physical engagement with the mesh assembly **124** and, in particular, a piezoelectric disk having a mesh material which, when powered by the power source, vibrates so as to aerosolize a liquid contained within the bladder **120** and conveyed in the wick **122.** The mesh assembly **124** is held in tension on top of a lip of the mouth of the bladder **120** by a sealing O-ring (see, e.g., FIG. 21, O-ring **212**) that is forced into engagement with the mesh assembly **124** by the attachment of the mouthpiece **108.**

Screws are preferably utilized in effecting the attachment whereby the force by which the O-ring is held in contact with the mesh assembly may be adjusted. A spacer on a printed circuit board of the cartridge assembly may additionally engage the bottom of the silicone bladder and hold the wick in tension therethrough. Due to these features, it is believed that the bladder and wick ensure that the mesh remains in constant contact with the liquid for consistent aerosolization each time the electronic device is triggered. The liquid preferably is supplied to the vibrating mesh at a generally constant pressure whereby a generally uniform aerosol is produced, and this is accomplished regardless of the orientation of the electronic device.

During a preferred manufacture of the disposable cartridge assembly, the bladder is filled with a liquid by injection after assembly of the disposable cartridge assembly. Since the bladder preferably is a self-sealing silicone bladder, when the injector needle is removed, the bladder re-seals and no liquid drains or leaks out. In this aspect, the liquid may be injected as a last stop via an access port/injector port that is located on the bottom of the cartridge. Alternatively, the bladder is inserted into the cartridge and then is filled with liquid first (top-down pour) without utilizing a needle or puncturing the bladder with an injector needle. In this manner, the bladder is filled by pouring liquid into the bladder and, once the desired volume has been dispensed, the wick is inserted inside the bladder and then the bladder is capped off by the mesh assembly and the rest of the disposable cartridge assembly is then assembled.

The handheld base assembly **102** comprises circuitry; and a power supply for actuating the mesh assembly **124** when the base assembly **102** and cartridge assembly **104** are coupled together, preferably through the pins that engage electrical contacts when the base assembly **102** and cartridge assembly **104** are coupled. One or more of the pins further are provided for electronic communication between circuitry of the handheld base assembly **102** and non-transitory machine-readable memory located within the cartridge assembly **104.**

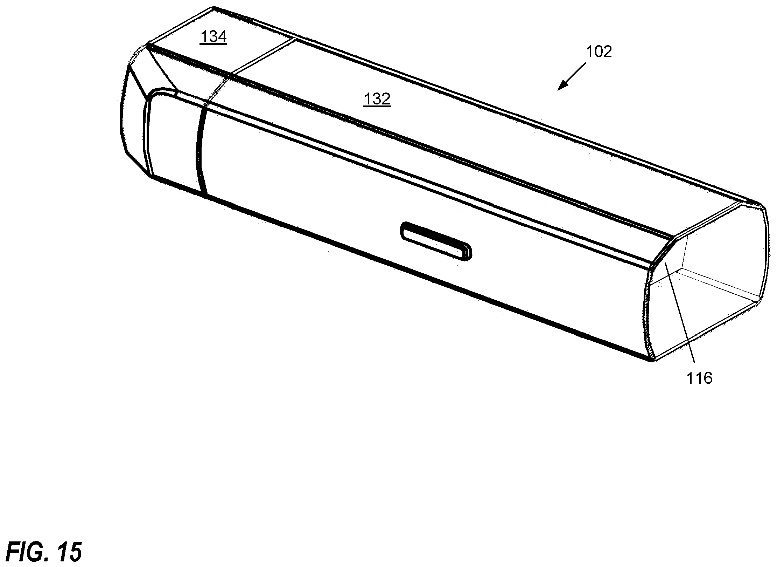

FIG. 15 is a perspective view of the handheld base assembly **102** of FIG. 1B.

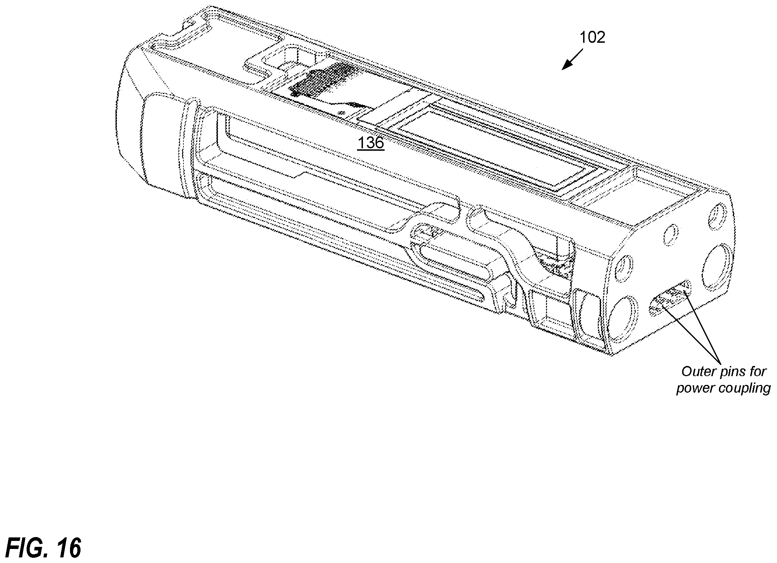

FIG. 16 is the perspective view of the handheld base assembly **102** of FIG. 15, wherein the skin **132** and tail **134** are omitted.

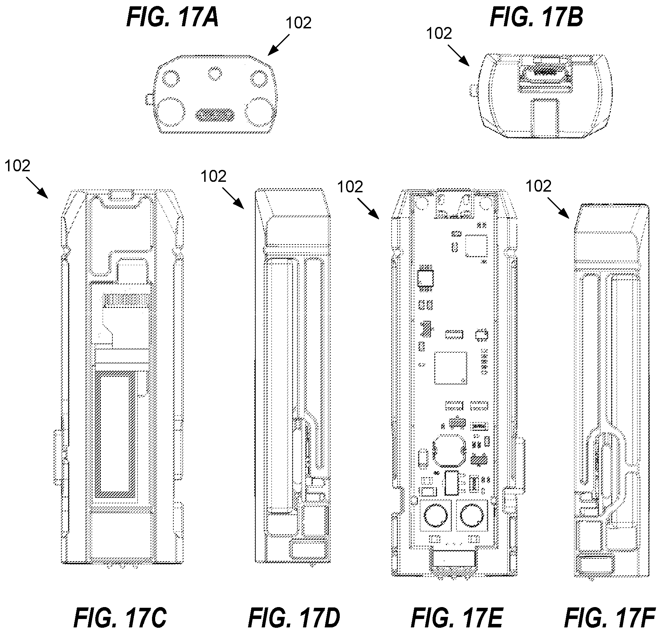

FIG. 17A is a front elevational view of the handheld base assembly **102** of FIG. 16; FIG. 17B is a rear elevational view of the handheld base assembly **102** of FIG. 16; FIG. 17C is a top plan view of the handheld base assembly **102** of FIG. 16; FIG. 17D is an elevational view of a first side of the handheld base assembly **102** of FIG. 16; FIG. 17E is a bottom plan view of the handheld base assembly **102** of FIG. 16; and FIG. 17F is an elevational view of a second, opposite side of the handheld base assembly **102** of FIG. 16.

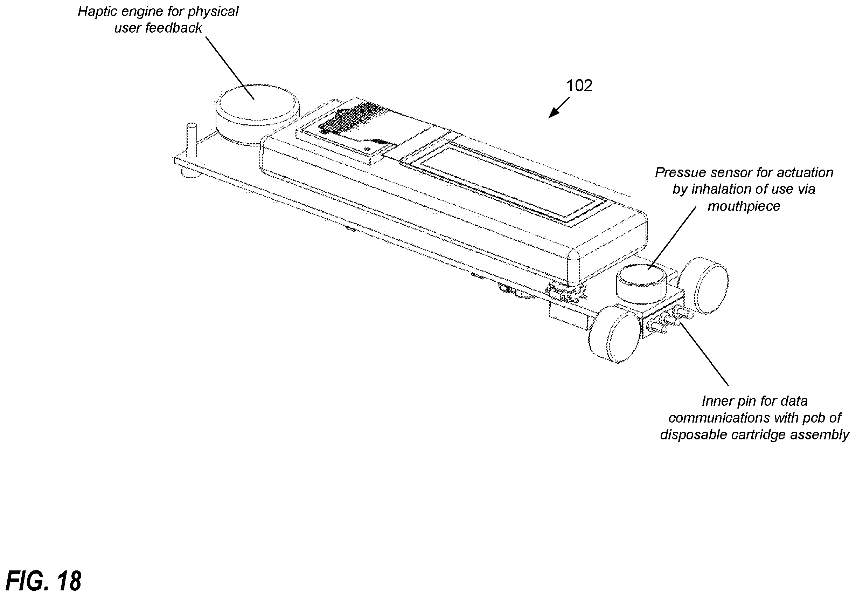

FIG. 18 is the perspective view of the handheld base assembly **102** of FIG. 16, wherein the chassis **136** is further omitted.

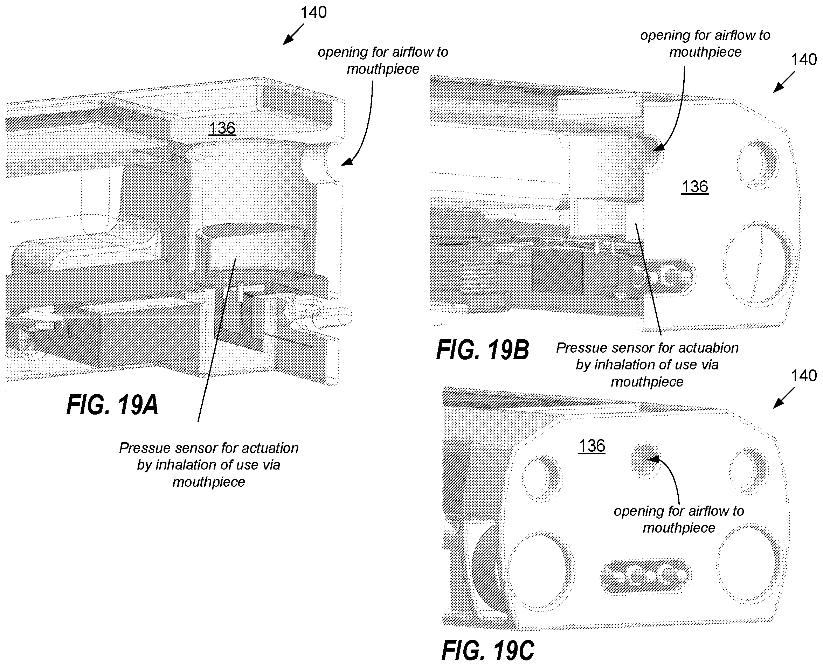

FIG. 19A is a partial, cross-sectional view of a distal end **140** of the front of the handheld base assembly of FIG. 16; FIG. 19B is another partial, cross-sectional view of the distal end **140** of the front of the handheld base assembly of FIG. 16; and FIG. 19C is a partial view of the distal end **140** of the front of the handheld base assembly of FIG. 16 similar to that of FIG. 19 B, but not in cross-section.

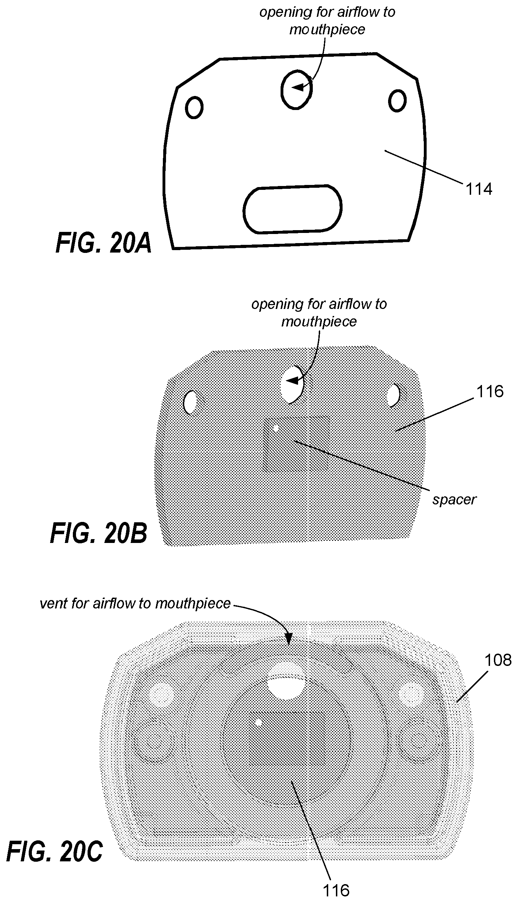

FIG. 20A is a perspective view of the metal plate **114** of FIG. 6B; FIG. 20B is a perspective view of the printed circuit board 116 of FIG. 7A; and FIG. 20C is a front elevational view of the cartridge **108** and printed circuit board **116,** wherein the cartridge **108** is transparently shown.

FIG. 21 is a view of disassembled components of a cartridge assembly **204** of a prototype commercial embodiment **200** of the invention.

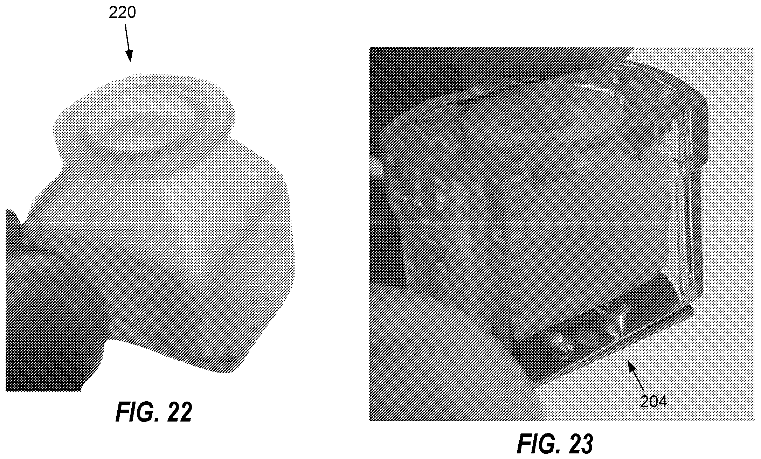

FIG. 22 is a perspective view of the bladder **220** of the cartridge assembly **204** of FIG. 21.

FIG. 23 is a perspective view of the bladder **220** of FIG. 22 positioned within the cartridge **204** of FIG. 21.

With regard to a prototype commercia embodiment, FIG. 24A is an elevational view of a top of the prototype commercial embodiment **200.** Additionally, FIG. 24B is an elevational view of a top of a prototype commercial embodiment **200,** wherein "WAVE" is being displayed on a display; and FIG. 24C is an elevational view of the top of the prototype commercial embodiment **200,** wherein a dose count of 97 is being displayed on the display. FIG. 24D is a perspective partial view of the prototype commercial embodiment **200** while a vapor is emitted from the mouthpiece. FIG. 24E is a perspective view of the prototype commercial embodiment **200** as an upward force is applied to the cartridge assembly **204** using two fingers while the base assembly **202** is held by the remaining fingers of a hand. FIG. 24F is a perspective view of the prototype commercial embodiment **200** following the application of the upward force of FIG. 24E, wherein the cartridge assembly **204** has been decoupled and removed from the handheld base assembly **202.** FIG. 24G is a view of the prototype commercial embodiment **200,** wherein the decoupled cartridge assembly **204** has been inverted to show a bottom thereof.

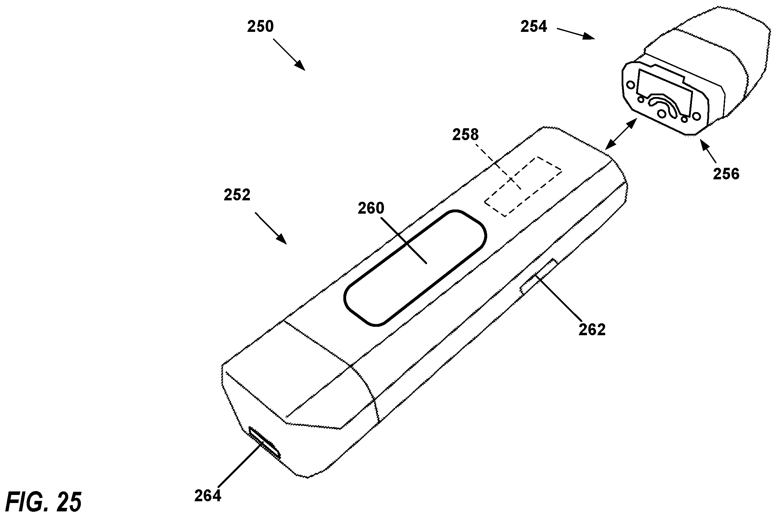

FIG. 25 is a perspective view of another prototype commercial embodiment intended for use in a preferred commercial system and apparatus for dosing by patients. Preferably, in such system and apparatus, the disposable cartridge assembly **254** of the electronic device **250** comprises a printed circuit board or other electronic circuitry **256,** and the disposable cartridge assembly communicates with electronic circuitry **258** contained in the handheld base assembly **250** when coupled therewith. Circuitry 258 preferably supports the capabilities of the disposable cartridge assembly as well as manages the OLED display **260,** recharging of the power source through USB port **264,** and operation of the button **262.**

Preferably, the printed circuit board or other electronic circuitry **256** includes non-transitory machine-readable memory, such as flash memory, that includes information regarding the liquid contained in the bladder and dosing information related thereto, e.g., the number of doses dispensed so far from the disposable cartridge assembly. The disposable cartridge assembly further may be configured or programmed to only work with one or more specified handheld base assemblies to the exclusion of other handheld base assemblies. In this respect, each handheld base assembly may include a unique identifier or other information, such as an encryption key, in non-transitory machine-readable memory, such as read-only memory, as part of the electronic circuitry **258.** Thus, for example, a disposable cartridge assembly could be configured to work only with a handheld base assembly of a particular person, e.g., a certain patient for whom a prescription is provided via the disposable cartridge assembly, to whom the handheld base assembly has been specifically programmed or configured. A plurality of disposable cartridge assemblies then may be provided to the patient over time as part of a subscription/prescription, in-home delivery system for continuity of care, especially in chronic disease management, wherein each disposable cartridge assembly is configured to work only with the handheld base assembly specifically programmed or configured for that specific patient and no other.

The electronic device **250** preferably comprises a breath-actuated, accurate, and efficient metered-dose delivery system. A haptic engine is provided for customizable haptic vibration to signal the end of precisely metered dose. The haptic feedback thus provides a biofeedback loop that preferably is customizable through a mobile app via wireless communication or communication, such as Bluetooth, or via the USB-port used for recharging the power source. The communications also provide a means for compliance check as well as provides accessible, real-time electronic medical record (EMR) data for providers, clinicians, and patients. Such communications further enable cartridge tracking, monitoring, user authentication, and geo-fencing capabilities for an increase standard of care and patience outcomes.

In the system using the electronic device **250** disclosed in FIG. 25, the patient's inhalation through a pressure sensor triggers the vibrating mesh to activate under normal use. Bluetooth-enabled mobile app integration logs precise dosing data in real-time, which is easily accessed by the patient and clinician. An OLED display visually indicates data to the patient including, for example, dose count, and displays intelligent prompts to the patient. The disposable cartridge assembly monitoring and memory capabilities further provide lifecycle, tamper-proof and chain of custody compliance from manufacture to delivery.

Based on the foregoing description, it will be readily understood by those persons skilled in the art that the invention has broad utility and application. Electronic devices of the invention can be utilized to deliver liquids comprising supplements, drugs, or therapeutically effective amounts of pharmaceuticals using an aerosol having particles of a size that can easily be inhaled. The aerosol can be used, for example, by a patient within the bounds of an inhalation therapy, whereby the liquid containing a supplement, therapeutically effective pharmaceutical, or drug reaches the patient's respiratory tract upon inhalation. Desired compounds such as nicotine, flavoring, and supplements like B12, can be received by a person through inhalation without the toxic byproducts like formaldehyde-a recognized Group 1 Carcinogen for caner-that is currently being created during heating in conventional vapes. Electronic devices of the invention further can be used in the marijuana industries, *but only where legal*, for delivery of cannabinoids and CBD oils and the like. Moreover, many embodiments and adaptations of the disclosure other than those specifically described herein, as well as many variations, modifications, and equivalent arrangements, will be apparent from or reasonably suggested by the invention and the foregoing descriptions thereof, without departing from the substance or scope of the invention.

It further will be appreciated from the foregoing that at least some preferred embodiments of the invention represent a portable, orientation-agnostic vibrating mesh nebulizer. It further will be appreciated from the foregoing that at least some preferred embodiments emit an aerosol that is-sensorially speaking-equivalent to vapor, i.e., not a mist but instead that which is generated by traditional vapes, thereby providing an enjoyable consumer product for those who are accustomed to vaping.

Accordingly, while the invention has been described herein in detail in relation to one or more preferred embodiments, it is to be understood that this disclosure is only illustrative and exemplary of the invention and is made merely for the purpose of providing a full and enabling disclosure of the invention.

## Claims

1. An electronic device (100) for producing an aerosol for inhalation by a person, comprising:
a cartridge assembly (104); and
a handheld base assembly (102);
wherein the cartridge assembly and handheld base assembly are configured to removably couple together;
wherein the cartridge assembly comprises:
a mouthpiece (106),
a cartridge (108), and
a bladder assembly (118); and
wherein the bladder assembly comprises:
a bladder (120),
a wick (122) contained within the bladder, and
a mesh assembly (124); and
wherein the mesh assembly comprises a mesh material and a piezoelectric material, the mesh material being configured to vibrate when the piezoelectric material is actuated whereby an aerosol is produced when the mesh material contacts a liquid of the bladder such that the aerosol may be inhaled through the mouthpiece.

2. The electronic device (100) of claim 1, wherein the handheld base assembly (102) comprises circuitry and a power supply for actuating the mesh assembly.

3. The electronic device (100) of claim 1, wherein the cartridge assembly (104) and handheld base assembly (102) are configured magnetically couple together.

4. The electronic device (100) of claim 1, wherein the cartridge assembly (104) magnetically mounts onto an end of the handheld base assembly.

5. The electronic device (100) of claim 1, wherein the mesh assembly (124) comprises a piezo mesh disk.

6. The electronic device (100) of claim 1, wherein the wick (122) comprises a lengthwise channel.

7. The electronic device (100) of claim 1, wherein the mesh assembly (124) is disposed on top of a lip of a mouth of the bladder (120), the bladder extending through an opening in the cartridge (108) to define the mouth.

8. The electronic device (100) of claim 1, wherein the wick (122) is retained in constant contact with the mesh assembly.

9. The electronic device (100) of claim 1, wherein an end of the wick (122) is secured by protuberances extending from walls proximate a bottom of the bladder.

## Patentansprüche

1. Elektronische Vorrichtung (100) zum Erzeugen eines Aerosols zur Inhalation durch eine Person, umfassend:
eine Kartuschenanordnung (104); und
eine tragbare Basisanordnung (102);
wobei die Kartuschenanordnung und die tragbare Basisanordnung dazu konfiguriert sind, sich entfernbar miteinander zu koppeln; wobei die Kartuschenanordnung Folgendes umfasst:
ein Mundstück (106),
eine Kartusche (108), und
eine Blasenanrodnung (118); und
wobei die Blasenanordnung Folgendes umfasst:
eine Blase (120),
einen Docht (122), der in der Blase enthalten ist, und
eine Maschenanordnung (124); und
wobei die Maschenanordnung ein Maschenmaterial und ein piezoelektrisches Material umfasst, wobei das Maschenmaterial dazu konfiguriert ist, zu vibrieren, wenn das piezoelektrische Material betätigt wird, wodurch ein Aerosol erzeugt wird, wenn das Maschenmaterial eine Flüssigkeit der Blase so berührt, dass das Aerosol durch das Mundstück inhaliert werden kann.

2. Elektronische Vorrichtung (100) nach Anspruch 1, wobei die tragbare Basisanordnung (102) Schaltungen und eine Stromversorgung zum Betätigen der Maschneanordnung umfasst.

3. Elektronische Vorrichtung (100) nach Anspruch 1, wobei die Kartuschenanordnung (104) und die tragbare Basisanordnung (102) magnetisch miteinander gekoppelt konfiguriert sind.

4. Elektronische Vorrichtung (100) nach Anspruch 1, wobei die Kartuschenanordnung (104) magnetisch an einem Ende der tragbaren Basisanordnung montiert ist.

5. Elektronische Vorrichtung (100) nach Anspruch 1, wobei die Maschenanordnung (124) eine Piezomaschenscheibe umfasst.

6. Elektronische Vorrichtung (100) nach Anspruch 1, wobei der Docht (122) einen längsgerichteten Kanal umfasst.

7. Elektronische Vorrichtung (100) nach Anspruch 1, wobei die Maschenanordnung (124) auf einer Lippe eines Mundes der Blase (120) angeordnet ist, wobei sich die Blase durch eine Öffnung in der Kartusche (108) erstreckt, um den Mund zu definieren.

8. Elektronische Vorrichtung (100) nach Anspruch 1, wobei der Docht (122) in ständigem Kontakt mit der Maschenanordnung gehalten wird.

9. Elektronische Vorrichtung (100) nach Anspruch 1, wobei ein Ende des Dochts (122) durch Vorsprünge gesichert ist, die sich von Wänden in der Nähe eines Bodens der Blase erstrecken.

## Revendications

1. Dispositif électronique (100) permettant de produire un aérosol destiné à être inhalé par une personne, comprenant :
un ensemble cartouche (104) ; et
un ensemble base portatif (102) ;
ledit ensemble cartouche et ledit ensemble base portatif étant conçus pour se coupler ensemble de manière amovible ;
ledit ensemble cartouche comprenant :
un embout buccal (106),
une cartouche (108), et
un ensemble vessie (118) ; et
ledit ensemble vessie comprenant :
une vessie (120),
une mèche (122) contenue dans la vessie, et
un ensemble maille (124) ; et
ledit ensemble maille comprenant un matériau maille et un matériau piézoélectrique, le matériau maille étant conçu pour vibrer lorsque le matériau piézoélectrique est actionné, grâce à quoi un aérosol est produit lorsque le matériau de maille entre en contact avec un liquide de la vessie de sorte que l'aérosol puisse être inhalé par l'embout buccal.

2. Dispositif électronique (100) selon la revendication 1, ledit ensemble de base portatif (102) comprenant des circuits et une alimentation électrique pour actionner l'ensemble maille.

3. Dispositif électronique (100) selon la revendication 1, ledit ensemble cartouche (104) et ledit ensemble base portatif (102) étant conçus pour être couplés magnétiquement l'un à l'autre.

4. Dispositif électronique (100) selon la revendication 1, ledit ensemble cartouche (104) étant monté magnétiquement sur une extrémité de l'ensemble base portatif.

5. Dispositif électronique (100) selon la revendication 1, ledit ensemble maille (124) comprenant un disque à maille piézoélectrique.

6. Dispositif électronique (100) selon la revendication 1, ladite mèche (122) comprenant un canal longitudinal.

7. Dispositif électronique (100) selon la revendication 1, ledit ensemble maille (124) étant disposé au-dessus d'une lèvre d'une bouche de la vessie (120), la vessie s'étendant à travers une ouverture dans la cartouche (108) pour définir la bouche.

8. Dispositif électronique (100) selon la revendication 1, ladite mèche (122) étant maintenue en contact constant avec ledit ensemble maille.

9. Dispositif électronique (100) selon la revendication 1, une extrémité de la mèche (122) étant fixée par des protubérances s'étendant depuis des parois proches du fond de la vessie.
